# EUROPEAN PATENT APPLICATION

(11) **EP 2 005 904 A2**
(43) Date of publication of application: **24.12.2008**
(21) Application number: 08158536.6
(22) Date of filing: 19.06.2008
(51) Int. Cl.: A61B 17/11

(54) **System for insertion of a graft**

(30) Priority: 19.06.2007 US 929248 P
(71) Applicant: HDH Medical Ltd., 31254 Haifa (IL)
(72) Inventor: Waysbeyn, Igor, 32696 Haifa (IL); Kavounovski, Irina, 32696 Haifa (IL)
(74) Representative: Dr. Graf & Partner

(57) **Abstract**

The present invention may provide tools for carrying out a method for insertion of a graft, a method of use comprising gripping a tubular object (500) by a grip head of a first tool (100), grasping the object by a second tool (320), detaching the object from the first tool and inserting the tubular object into a tubular cavity, wherein when inserting, a longitudinal axis of the second tool is substantially perpendicular to a longitudinal axis of the tubular cavity.

## Description

### BACKGROUND OF THE INVENTION

Known methods and tools for insertion of a graft to and placement in a vessel are not specifically designed for the laparoscopic surgery and therefore may be clumsy and inconvenient.

Some known methods include grasping and bending a vessel in a convenient direction for insertion of a graft. These methods may stretch and damage the vessel.

Some known methods include gripping the graft, for example, by forceps or tweezers, and the gripping may not be firm enough so that, for example, the graft may slip. Additionally, the direction in which the graft may be held may change because the grip may include only two hubs of contact.

Holding a graft by regular forceps, for example so that the longitudinal axis of the forceps is substantially parallel to the longitudinal axis of the graft, in some cases may compel the user to push the graft into the vessel diagonally, thus stretching the vessel and possibly causing damage to the living tissue.

Other tools and methods may be inconvenient and not be suitable for insertion into the body through a trocar or other device or incisions, as usually done in laparoscopic processes. Some tools and methods may be usable only in open surgeries.

Therefore there is a need for method and tools designed especially for convenient use in implantation of a graft in a vessel, particularly in laparoscopic surgeries.

### BRIEF DESCRIPTION OF THE DRAWINGS

The subject matter regarded as the invention is particularly pointed out and distinctly claimed in the concluding portion of the specification. The invention, however, both as to organization and method of operation, together with objects, features, and advantages thereof, may best be understood by reference to the following detailed description when read with the accompanying drawings in which:

Fig. 1A is a schematic illustration of a delivery tool according to embodiments of the present invention;

Fig. 1B is a schematic cross-sectional illustration of a delivery tool according to embodiments of the present invention;

Fig. 1C is a schematic three-dimensional illustration of a grip head of a delivery tool according to embodiments of the present invention;

Fig. 1D is a schematic three-dimensional and partially transparent illustration of a grip head gripping an object according to embodiments of the present invention;

Fig. 2A is a schematic illustration of a measurement tool according to embodiments of the present invention;

Figs. 2B and 2C are schematic illustrations of a measurement tool from two different angles according to embodiments of the present invention, wherein Fig. 2C is cross sectional;

Fig. 2D is a schematic three dimensional illustration of a portion of a measurement tool according to embodiment of the present invention;

Figs. 2E and 2F are schematic illustrations of two states of a flexible stripe of a measurement tool during insertion and/or extraction of the measurement tool through a pipe according to embodiments of the present invention;

Figs. 3A and 3B are schematic illustrations of portions of a measurement tool according to embodiment of the present invention;

Fig. 3C is a schematic cross-sectional illustration of a portion of a measurement tool according to embodiment of the present invention;

Figs. 4A and 4B are schematic illustrations of an insertion tool according to embodiments of the present invention;

Figs. 4C and 4D are schematic three-dimensional illustrations of two positions of an insertion tool according to embodiments of the present invention;

Fig. 4E is a schematic cross sectional illustration of a grasping tip and stabilizing tips of an insertion tool grasping a tubular fastener according to embodiments of the present invention; and

Fig. 5 is a flow chart describing a method for insertion of a graft according to embodiments of the preset invention.

It will be appreciated that for simplicity and clarity of illustration, elements shown in the figures have not necessarily been drawn to scale. For example, the dimensions of some of the elements may be exaggerated relative to other elements for clarity. Further, where considered appropriate, reference numerals may be repeated among the figures to indicate corresponding or analogous elements.

### DETAILED DESCRIPTION OF THE PRESENT INVENTION

In the following detailed description, numerous specific details are set forth in order to provide a thorough understanding of the invention. However, it will be understood by those skilled in the art that the present invention may be practiced without these specific details. In other instances, well-known methods, procedures, and components have not been described in detail so as not to obscure the present invention.

Embodiments of the present invention may provide a method for easy and safe insertion of a graft to vessel with minimal potential damage to the living tissue and to the graft. A set of tools enabling the method may be also provided.

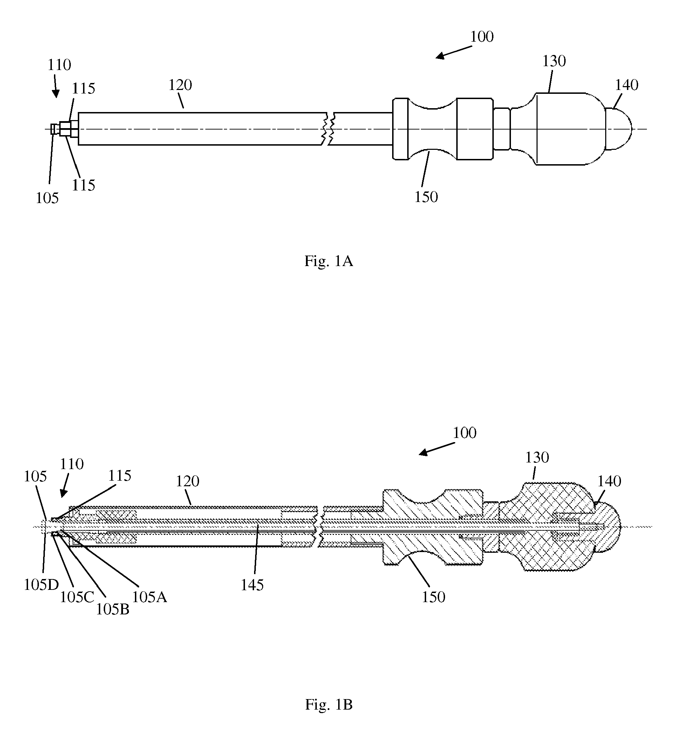

Reference is made to Fig. 1A, which is a schematic illustration of delivery tool 100 according to embodiments of the present invention. Delivery tool 100 may be insertable into a body lumen preferably through a trocar or other device or incisions (not shown) commonly used in laparoscopic surgery. Delivery tool 100 may include a handhold 150, a handle 130, control means 140, a tube 120 and grip head 110. Delivery tool 100 may be held by handhold 150 and thus, for example, be directed inside the body lumen. Grip head 110 may grip a tubular object, for example by adjustment of an external diameter of grip head 110, at a certain position on the grip head, to an internal diameter of the tubular object until a firm grip is achieved. Grip head 110 may include head segments 115, which may draw near and away from each other, thus, for example, changing the external diameter of grip head 110. Grip head 110 may include adjustment head 105 which may controllably adjust the external diameter of grip head 110. Adjustment head 105 may include varying external diameter and thus, for example, may change the external diameter of grip head 110 by insertion of the suitable portion of adjustment head 105 between head segments 115. Adjustment head 105 may have, for example, a conical shape. Preferably, the external diameter of adjustment head 105 may get larger in a direction from the tool outwards. Adjustment head 105 and/or the external diameter of grip head 110 may be controllable by control means 140. For example, pushing of control means 140 may cause reduction of the external diameter of grip head 110, thus, for example, releasing a tubular body from grip by grip head 110. Similarly, pulling of control means 140 may cause insertion of a portion of adjustment head 105 between head segments 115, thus, for example, expanding the external diameter of grip head 110 correspondingly to the external diameter of the inserted portion of adjustment head 105. Control means 140 may include, for example, a spring apparatus (not shown). For example, when released, control means 140 may expand the external diameter of grip head 110 to its maximum size and/or until it meets an internal diameter of another object to be gripped by grip head 110. The grip of the object may be secured by the constant force of the spring. By pushing control means 140, the external diameter of grip head 110 may be changed, for example, reduced.
When an object is gripped by grip head 110, reducing of the external diameter of grip head 110 may release the grip and the object may be detached from delivery tool 100.

Reference is made to Fig. 1B, which is a schematic cross-sectional illustration of delivery tool 100 according to embodiments of the present invention. As described above, adjustment head 105 may be controlled by control means 140. Control means 140 may control adjustment head 105 through a control shaft 145. As described above, adjustment head 105 may include several portions with different external diameters, for example, portions 105A-105D. Each of portions 105A-105D may have a different external diameter. Preferably, the external diameter may get larger as the distance of the portion from control shaft 145 is larger. In the example of Fig 1B, portion 105B may have a greater external diameter than portion 105A, portion 105C may have a greater external diameter than portion 105B and portion 105D may have a greater external diameter than portion 105C. The external diameter of grip head 110 may be changed correspondingly to the external diameter of the portion of adjustment head 105 inserted within grip head 110, for example, by pushing apart head segments 115.

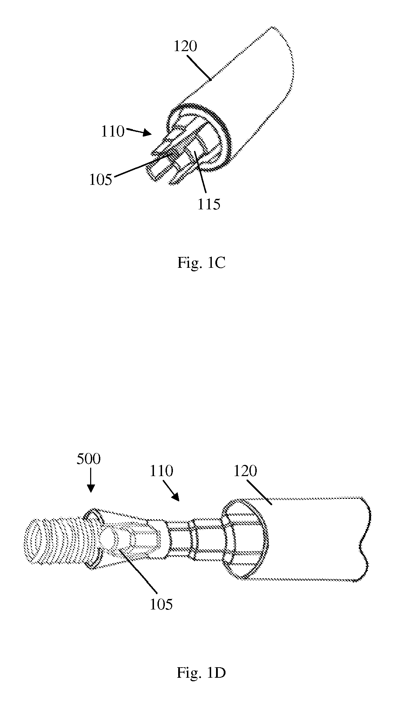

Reference is made to Fig. 1C, which is a schematic three-dimensional illustration of grip head 110 according to embodiments of the present invention. As described above, head segments 115 may be pushed apart, thus, for example, expanding the external diameter of grip head 110. Accordingly, the external diameter of grip head 110 may be changed correspondingly to the external diameter of the adjustment head 105 which may be inserted in between head segments 115.

Reference is made to Fig. 1D, which is a schematic three-dimensional and partially transparent illustration of grip head 110 gripping an object 500 according to embodiments of the present invention. Grip head 110 may include terraced external diameter, for example in order to match different internal diameters of different objects. Further adjustment of the external diameter of grip head 110 may be achieved for example, by controlling adjustment head 105 as described in detail above with reference to Figs. 1A-1C. The terraced external diameter of grip head 110 and/or the adjustment by adjustment head 105 may enable, for example, firm grip of object 500 on grip head 110. Grip head 110, with or without griped object 500, may be pulled and/or inserted into tube 120. The insertion and/or extraction of grip head 110 into and/or from tube 120 may be controlled by handle 130 which may move relatively to handhold 150, along a longitudinal axis of delivery tool 100. For example, grip head 110, with or without griped object 500, may be stowed within tube 120 during the insertion of delivery tool 100 into a body lumen, and may be pushed out of tube 120 when inside a body lumen. The insertion of grip head 110 into tube 120 during the insertion of delivery tool 100 into a body lumen may prevent grip head 110 from being stuck and/or damaged, for example, by a valve installed in the trocar, which may prevent or delay the insertion of delivery tool 100 into a body lumen and/or damage delivery tool 100 and/or the trocar. The insertion of grip head 110 into tube 120 during the insertion of delivery tool 100 into a body lumen may also protect object 500 from hitting other objects, such as the valve of the trocar and external tissues like skin, muscles, intestines and others.

The internal diameter and/or length of tube 120, if known, may be used also for evaluating the size of object 500 and thus, for example, whether it may fit a vessel or other body to which it may be inserted.

Delivery tool 100 may be in any required size in order to match a range of sizes of grafts or other tubular objects to be griped. In some embodiments of the present invention, a measurement may be performed inside a body lumen in order to choose, for example, the right size of a graft and/or the right size of the tools.

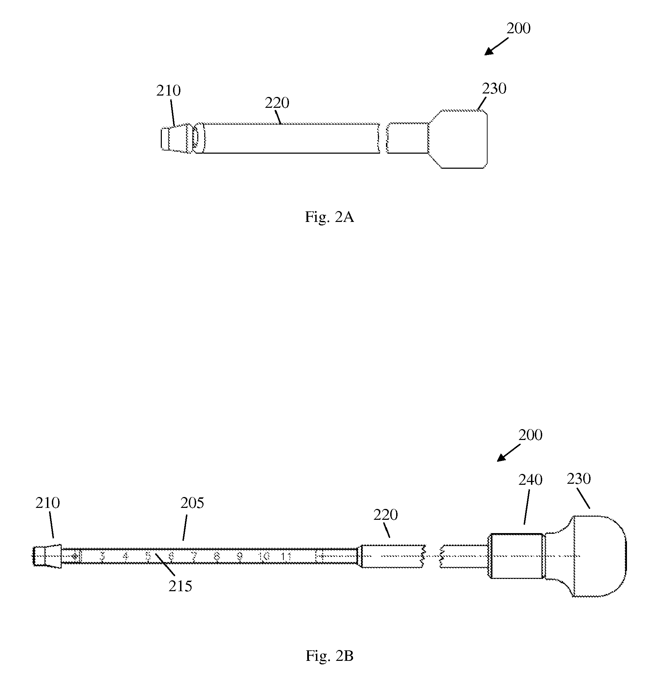

Reference is now made to Fig. 2A, which is a schematic illustration of measurement tool 200 according to embodiments of the present invention. Measurement tool 200 may be insertable into a body lumen preferably through a trocar commonly used in laparoscopic surgery. Measurement tool 200 may include a handhold 240, handle 230, tube 220 and measurement head 210. Measurement head 210 may be used for diameter measurement, for example of a vessel. According to this measurement, the suitable size of a graft, optionally attached to a fastener, to be inserted to the vessel may be chosen. Accordingly, the suitable size of delivery tool 100 may be chosen. In order to enable measurement in different directions, measurement tool 200 may include a flexible stripe as will be shown in the following figures. In order to measure length, measurement tool 200 may also include a ruler on the flexible stripe (shown, for example, in Fig. 2B). As will be described in detail herein below, the flexible stripe may be controllably inserted into or taken out of tube 220, for example, by handle 230. Measurement tool 200 may be held by handhold 240 and thus, for example, be directed inside the body lumen. Handle 230 may move relatively to handhold 240, for example, along a longitudinal axis of measurement tool 200, thus, for example, controlling measurement head 210 and stripe 205. For example, the length of stripe 205 extending out of tube 220 may be controlled by Handle 230.

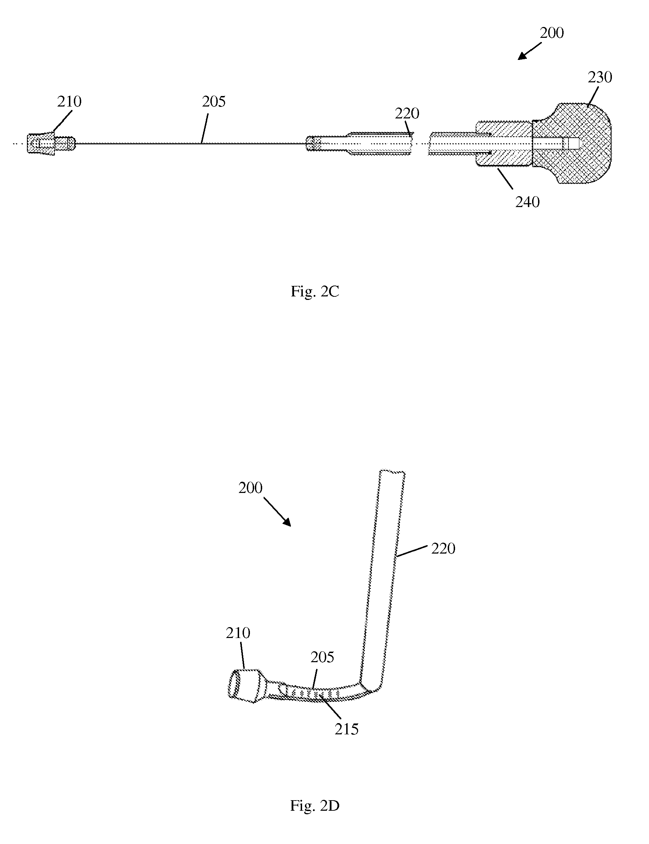

Reference is now made to Figs. 2B and 2C, which are schematic illustrations of measurement tool 200 from two different angles according to embodiments of the present invention. Fig. 2C is a cross sectional illustration. As mentioned herein above, measurement tool 200 may include a flexible stripe 205. Measurement head 210 may be located at the distal end of flexible stripe 205. Flexible stripe 205 may be controllably inserted or taken out of tube 220, for example, by handle 230. Flexible stripe 205 may enable length measurements inside the body lumen by placing the required portion of stripe 205 against or next to the organ to be measured, for example, by including a ruler 215. According to the length measurement, for example, a graft with the suitable length may be prepared outside of the body and, inserted into the body lumen and placed in a vessel. Other objects and/or tools may be chosen or prepared based on the length measurement.

Reference is now made to Fig. 2D, which is a schematic three dimensional illustration of a portion of measurement tool 200 according to embodiment of the present invention. Flexible stripe 205 may controllably bend in directions perpendicular to the plane of its face while resisting bending in other directions, for example, according to a force vector provided by a user applied at the distal end of tube 220. The bending may enable measurement in different directions by measurement head 210 and/or ruler 215. By choosing the desired length of stripe 205 extending out of tube 220 the pressure needed at the measurement head 210, for example, in order to bend stripe 205, may be controlled, thus providing another degree of freedom for the user to operate measurement tool 200.

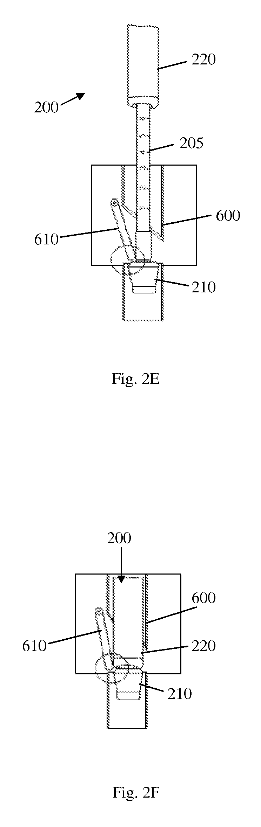

As described above, flexible stripe 205 may be controllably inserted or taken out of tube 220, for example, by handle 230. For example, flexible stripe 205 may be within tube 220 during the insertion or extraction of measurement tool 200 into or from a body lumen, and may be pushed out of tube 220 when inside a body lumen. Reference is now made to Figs. 2E and 2F, which are schematic illustrations of two states of flexible stripe 205, during insertion or extraction of measurement tool 200 through a pipe 600 according to embodiments of the present invention. Pipe 600 may be, for example, a trocar commonly used in laparoscopic procedures. Pipe 600 may include a valve 610 which may, for example, limit transition of air and/or other gases or fluids out of the body lumen. As shown in Fig. 2E, flexible stripe 205 may be out of tube 220 during insertion or extraction of measurement tool 200 through a pipe 600. In this case, valve 610 may prevent or delay the insertion or extraction of measurement tool 200 into or from a body lumen and/or damage measurement tool 200 and/or pipe 600. As shown in Fig. 2F, flexible stripe 205 may be stowed within tube 220 during the insertion or extraction of measurement tool 200, forming a substantially continuous outer line of tube 220 with measurement head 210 and thus, for example, preventing measurement tool 200 from being stuck, for example, by valve 610.

In some cases, the size of measurement head 210 may be too big in order to be inserted through a trocar such as pipe 600 shown in Figs. 2E and 2F. For example, the trocar diameter may be of about 10-15 mm while the diameter of the vessel may be of about 20-30 mm. In some embodiments of the present invention, a measurement tool similar to measurement tool 200 may include an inflatable measurement head instead of measurement head 210. The measurement tool may be inserted through the trocar in its deflated mode and to be inflated to a required size inside the body after being inserted through the trocar.

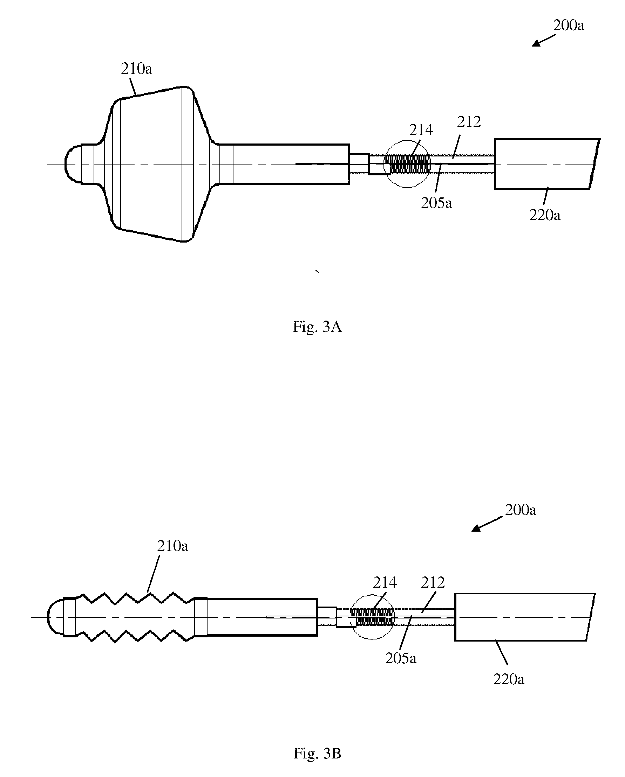

Reference is now made to Figs. 3A and 3B, each of which is a schematic illustration of a portion of a measurement tool 200a according to embodiment of the present invention. Measurement tool 200a may include a tube 220a and a flexible stripe 205a. Tube 220a and flexible stripe may operate similarly to tube 220 and flexible stripe 205 discussed above with reference to Figs 2A-2F. Flexible stripe 205a may include a hollow flexible tube 212 along a longitudinal axis of stripe 205a. In Figs. 3A and 3B flexible stripe 205a is shown in a side view so that stripe 205a looks like a narrow line and tube 212 extends beyond the narrow line in both sides. Flexible tube 212 may be surrounded by a spring 214 twisted around it, for example, in order to reinforce and/or keep tube 212 from breaking, for example, when flexible stripe 205a is bending, thus, for example, allowing tube 212 to bend together with flexible stripe 205a. Additionally, spring 214 may be attached to flexible stripe 205a, thus, for example, holding tube 212 and flexible stripe 205a together.

Measurement tool 200a may include an inflatable measurement head 210a. When inflatable measurement head 210a is inflated, as shown in Fig. 3A, it may obtain an inflated target shape and may operate similarly to measurement head 210 as discussed above with reference to Figs. 2A-2F. Measurement head 210a may be inflatable to a predetermined target size and/or shape. Measurement head 210a with a specific predetermined inflated size may be selected by a user according to the estimated size of the vessel to be operated. The selected head 210a may be installed on tool 200a. Inflatable head 210a may be made of, for example, polyamide compound, polyvinyl chloride (PVC), cross-linked polyethylene (PE), polyethylene terephthalate (PET), nylon, Nylon elastomers, Polyurethane, and/or other thermoplastic elastomers or a combination thereof. Inflatable head 210a may have a predetermined target size and/or shape. There are various methods for production of inflatable objects with a predetermined target size and/or shape. Some of these methods may include, for example, blow molding.

Measurement tool 200a may be inserted through a trocar into a body lumen, for example, a trocar similar to pipe 600 as shown in Figs. 2E and 2F. Inflatable measurement head 210a may be inserted through the trocar in its deflated mode, as shown in Fig. 3B. This way, for example, a measurement head which has, when inflated, a diameter bigger than the diameter of the available pass through the trocar, may be inserted into the body lumen through the trocar.

Inflatable head 210a, when inflated, may be rigid enough for the measurement purpose, for example, in order to keep its form when inserted into the vessel in order to measure the size of the vessel. Inflatable head 210a may by inflated by injection of fluid into inflatable head 210a, until sufficient pressure is produced inside inflatable head 210a and/or until inflatable head 210a reaches a desired shape. The pressure of the fluid inside the inflatable head 210a when inflated may be, for example, around 1-2Atm.

The fluid may be provided to inflatable head 210a by a pipe passing through measurement tool 200a and through tube 212 inside the flexible stripe 205a.

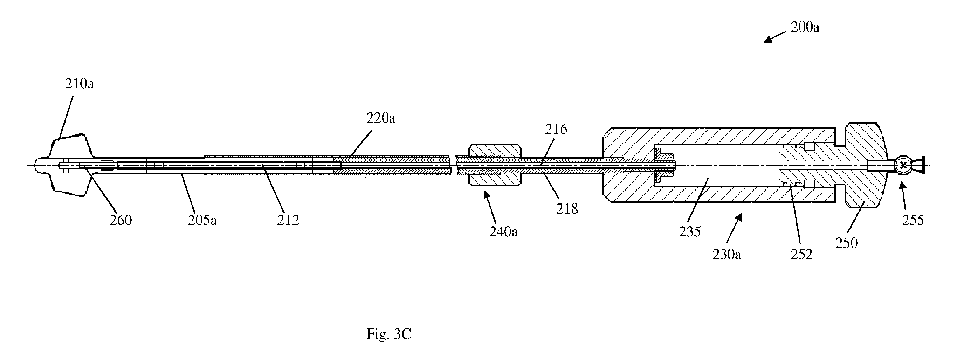

Reference is now made to Fig. 3C, which is a schematic cross-sectional illustration of a portion of measurement tool 200a according to embodiment of the present invention. Measurement tool 200a may include a handle 230a, which may include a spigot top 250, through which fluid may be provided into measurement tool 200a. Spigot top 250 may include a port 255, to which a fluid source (not shown) may be connected, for example, an injector. Handle 230a may further include a void 235. Tool 200a may further include a tube 218 with a channel 216 along its longitudinal axis. Tube 218 may be connected to handle 230a so that, for example, fluid provided into void 235 may flow into channel 216. Measurement tool 200a may further include a hold 240a, which may be connected to tube 220a which is described above with reference to Figs. 3A and 3B. Tube 218 may be threaded through hold 240a and tube 220a. Tube 218 may be slidable within hold 240a and tube 220a. Measurement tool 200a may further include flexible stripe 205a which is described above with reference to Figs. 3A and 3B. Flexible stripe 205a may be connected to tube 218 so that, for example, channel 216 may continue through flexible stripe 205a, for example as tube 212 or inside tube 212. Flexible stripe 205a may be connected in another side to inflatable head 210a. Inflatable head 210a may include a channel 260 which may conduct fluid which may arrive, for example, in channel 216 and/or tube 212, into head 210a.

In order to inflate head 210a, a fluid source, for example, an injector, may be connected to port 255 and, for example, provide fluid into void 235. The provided fluid may flow from void 235 to head 210a, for example, through channel 216 and/or tube 212. The pressure of the fluid inside tool 200a may be adjusted by spigot top 250. Spigot top 250 may include a piston 252, which may, for example, be pushed or pulled and/or screw-threaded in or out void 235. The pressure of the fluid inside tool 200a may be increased, for example, by pushing and/or screw-threading piston 252 into void 235, thus, for example, decreasing the volume of void 235 and increasing the pressure of the fluid inside tool 200a. The pressure of the fluid inside tool 200a may be decreased, for example, by pulling and/or screw-threading piston 252 out of void 235, thus, for example, increasing the volume of void 235 and decreasing the pressure of the fluid inside tool 200a.

Flexible stripe 205a may be controllably inserted or taken out of tube 220a, for example, by handle 230a. Flexible stripe 205a may enable length measurements inside the body lumen by placing the required portion of stripe 205a against or next to the organ to be measured, for example, by including a ruler (similar to ruler 215 of Figs 2B-2D). As described with reference to Figs. 2A-2F, according to the length measurement, for example, a graft with the suitable length may be prepared outside of the body and, inserted into the body lumen and placed in a vessel. Other objects and/or tools may be chosen or prepared based on the length measurement.

As described in detail above with regard to flexible stripe 205 of Figs. 2A-2F, flexible stripe 205a may controllably bend, for example, according to a force vector provided by a user applied at the distal end of tube 220a. The bending may enable measurement in different directions by measurement head 210a and/or flexible stripe 205a. By choosing the desired length of stripe 205a extending out of tube 220a the pressure needed at the measurement head 210a, for example, in order to bend stripe 205a, may be controlled, thus providing another degree of freedom for the user to operate measurement tool 200a.

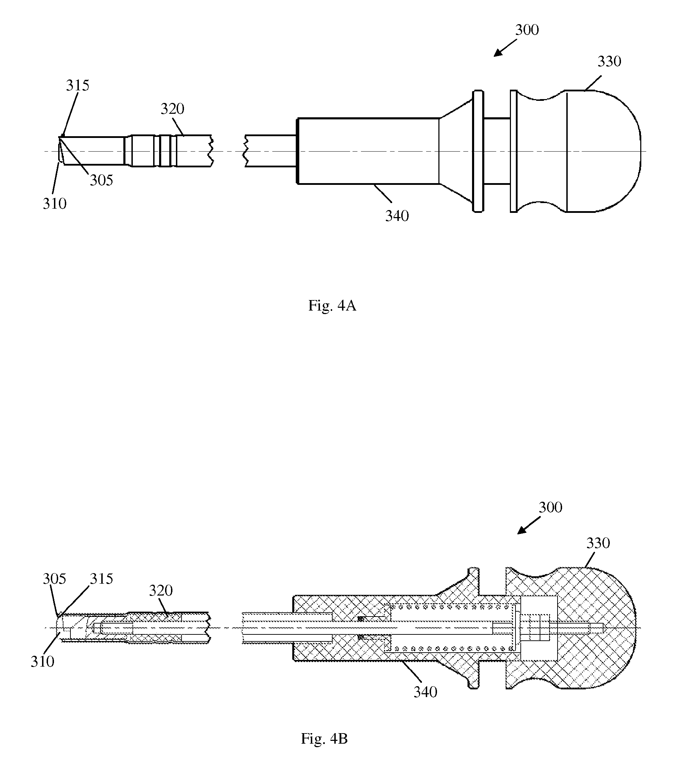

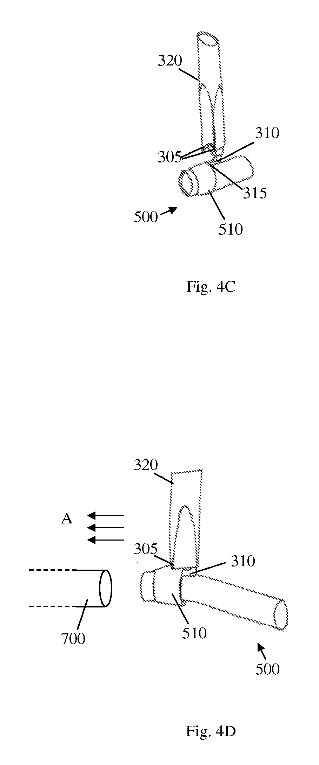

Embodiments of the present invention may enable convenient and easy method for insertion of a graft to a vessel, with minimum stretch and damage to the living tissue and/or to the vessel. Generally, the present invention may also be used for insertion of any suitable tubular object to another tubular cavity. Reference is now made to Figs. 4A and 4B, which are schematic illustrations of an insertion tool 300 according to embodiments of the present invention. Insertion tool 300 may include a handhold 340, a handle 330, a tube 320 and a grasping head 310. Insertion tool 300 may be held by handhold 340 and thus, for example, be directed inside the body lumen. Grasping head 310 may controllably be pushed or pulled away from tube 320 or to tube 320, respectively. Handle 330 may control grasping head 310 by moving relatively to handhold 340. Grasping head 310 may include a grasping tip 315. Insertion tool 300 may include stabilizing tips 305 arranged parallel to each other and to grasping tip 315. An object may be grasped between grasping tip 315 and stabilizing tips 305. As will be described in detail herein below with reference to Figs. 4C to 4E, grasping tip 315 and stabilizing tips 305 may be used for holding, for example, a fastener head connected to graft to be inserted to vessel.

Reference is made to Figs. 4C and 4D which are schematic three-dimensional illustrations of two positions of insertion tool 300 according to embodiments of the present invention. Grasping head 310 may be pushed away from tube 320, for example, in order to release a grasped object or before grasping an object. Grasping tip 315 may be inserted into the inner cavity of a tubular fastener 510, next to the inner perimeter of tubular fastener 510 encircling and connected to tubular object 500. For example, if the tubular object is a graft 500 to be inserted to vessel 700, tip 315 may be inserted into an inner cavity of a tubular fastener head 510, next to the inner perimeter of fastener head 510. Grasping head 310 may be pulled towards tube 320, thus, for example, tightening stabilizing tips 305 against the outer perimeter of tubular fastener 510. Preferably, insertion tool 300 may include two stabilizing tips 305, so that together with grasping tip 315 there may be provided three hubs of contact, known to be able to provide tight hold and self adjustment on the grasped object, for grasping tubular fastener 510. The three points of contact may prevent unwanted slips of insertion tool 300 and/or fastener 510 and may provide firm grasping of tubular fastener 510, which may enable safe steering of fastener 510 inside the body lumen and into a desired location in the vessel. When grasping tubular fastener 510, a longitudinal axis of insertion tool 300 may be substantially perpendicular to a longitudinal axis of tubular fastener 510. This position may provide a convenient approach for inserting tubular object 500 together with tubular fastener 510 into tubular cavity 700, for example, because longitudinal axis of insertion tool 300 may be substantially perpendicular to a longitudinal axis of tubular cavity 700. Insertion of tubular object 500 together with tubular fastener 510 into tubular cavity 700 may be achieved, for example, by merely moving insertion tool 300 in the general direction of arrows A.

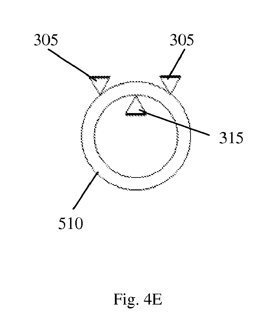

Reference is made to Fig. 4E, which is a schematic cross sectional illustration of grasping tip 315 and stabilizing tips 305 grasping tubular fastener 510 according to embodiments of the present invention. As described above, two stabilizing tips 305 together with grasping tip 315 may provide three hubs of contact for grasping tubular fastener 510. Since grasping tip 315 and stabilizing tips 305 have, actually, a longitudinal dimension perpendicular to the plane of Fig. 4E, the grasping provided by grasping tip 315 and stabilizing tips 305 has a an extra stability. The three hubs of contact may prevent unwanted slips of insertion tool 300 and may provide firm grasping of tubular fastener 510 and good level of steering of tubular fastener 510.

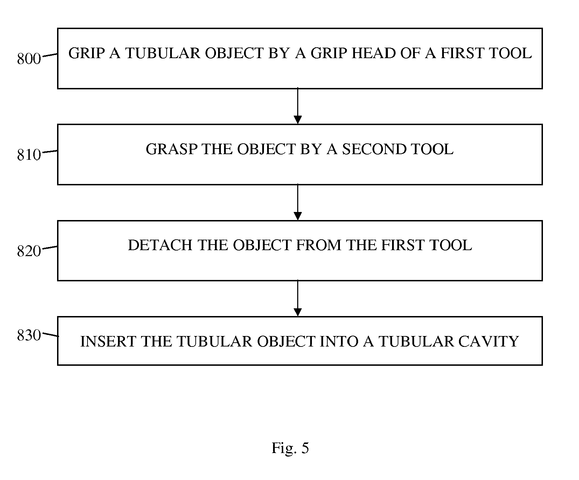

Reference is made to Fig. 5, which is a flow chart describing a method for insertion of a graft according to embodiments of the preset invention. The method may preferably be carried out by tools similar to the tools described above. As shown in block 800, the method may include gripping tubular object 500 by grip head 110 of delivery tool 100. As shown in block 810, the method may include grasping tubular object 500 by insertion tool 300. When grasping object 500, the longitudinal axis of insertion tool 300 may be substantially perpendicular to a longitudinal axis of tubular fastener 510. As shown in block 820, the method may include detaching tubular object 500 from delivery tool 100. As shown in block 830, the method may include inserting tubular object 500 into tubular cavity 700. When inserting tubular object 500 into tubular cavity 700, the longitudinal axis of insertion tool 300 may be substantially perpendicular to a longitudinal axis of tubular cavity 700.

While certain features of the invention have been illustrated and described herein, many modifications, substitutions, changes, and equivalents will now occur to those of ordinary skill in the art. It is, therefore, to be understood that the appended claims are intended to cover all such modifications and changes as fall within the true spirit of the invention.

## Claims

1. A system comprising:
a delivery tool for gripping a tubular object by a grip head;
an insertion tool for grasping said object so that a longitudinal axis of said insertion tool is substantially perpendicular to a longitudinal axis of a tubular fastener encircling said tubular object, and for inserting said tubular object into a tubular cavity,
wherein when inserting, a longitudinal axis of said insertion tool is substantially perpendicular to a longitudinal axis of said tubular cavity.

2. A system according to claim 1, wherein said grip head has a variable external diameter for gripping said tubular object by adjusting the external diameter of said grip head to an internal diameter of said tubular object, wherein the external diameter of said grip head is reducible to allow detaching of said tubular body from said grip head.

3. A system according to claim 1, wherein said insertion tool has a grasping head for grasping said tubular fastener by three hubs of contact and a handle for controlling the grasping by moving along a longitudinal axis of said insertion tool.

4. A system according to claim 1, further comprising a measurement tool for measuring size of said tubular cavity, said measurement tool comprising:
measurement head for measuring a diameter of a tubular cavity by insertion of said measurement head into said tubular cavity; and
a flexible stripe bendable in a desirable direction, wherein said measurement head is located at a distal end of said flexible stripe.

5. A system according to claim 4, wherein said flexible stripe comprises a ruler and/or is for measuring at least part of the length of said tubular cavity.

6. A system according to claim 4, wherein said measurement tool further comprises a tube, wherein said flexible stripe is insertible into said tube at least during passing of said measurement tool into a body lumen, and further comprises a handle for controlling the length of said flexible stripe extending out of said tube by moving along a longitudinal axis of said measurement tool.

7. A system according to claim 4, wherein the measurement head is an inflatable measurement head, said inflatable measurement head being in a deflated state at least during passing of said measurement tool into a body lumen and inflatable to a predetermined shape and/or size at least after insertion of said head into said body lumen

8. A system according to claim 1, wherein said grip head comprises at least two segments, said segments are for being departed from each other or for being brought closer to each other to modify the external diameter of said grip head, and wherein said delivery tool further comprises an adjustment head having a varying external diameter for adjusting the external diameter of said grip head by insertion or extraction of said adjustment head between segments of said grip head, wherein the external diameter of said grip head is reducible to allow detaching of said tubular body from said grip head.

9. A system according to claim 1, wherein said delivery tool further comprises a tube, wherein said grip head is insertible into said tube at least during passing of said delivery tool into a body lumen.

10. A system according to claim 1, wherein said delivery tool further comprises control means on a handle of said delivery tool for controlling gripping and detaching of said tubular object.

11. A delivery tool comprising:
a grip head having variable external diameter, for gripping a tubular object by adjusting the external diameter of said grip head to an internal diameter of said tubular object.

12. An insertion tool for inserting a tubular object into a tubular cavity, so that when inserting, a longitudinal axis of said insertion tool is substantially perpendicular to a longitudinal axis of said tubular cavity, the insertion tool comprising:
grasping head for grasping a tubular fastener encircling said tubular object, so that when grasping, a longitudinal axis of said insertion tool is substantially perpendicular to a longitudinal axis of said tubular fastener.

13. A measurement tool comprising:
measurement head for measuring a diameter of a tubular cavity by insertion of said measurement head into said tubular cavity; and
a flexible stripe bendable in a desirable direction, wherein said measurement head is located at a distal end of said flexible stripe.
